# EUROPÄISCHE PATENTSCHRIFT

(11) **EP 1 527 055 B1**
(45) Veröffentlichungstag und Bekanntmachung des Hinweises auf die Patenterteilung: **08.03.2006**
(21) Anmeldenummer: 03765085.0
(22) Anmeldetag: 22.07.2003
(51) Int. Cl.: C07D 301/12, B01D 3/14

(54) **VERFAHREN ZUR KONTINUIERLICHEN REINDESTILLATION DES BEI DER PROPYLENOXIDSYNTHESE VERWENDETEN LÖSUNGSMITTELS METHANOL**
METHOD FOR THE CONTINUOUS PURIFICATION BY DISTILLATION OF THE SOLVENT METHANOL, USED IN THE SYNTHESIS OF PROPYLENE OXIDE
PROCEDE DE PURIFICATION PAR DISTILLATION EN CONTINU DU METHANOL, SOLVANT EMPLOYE POUR LA SYNTHESE D'OXYDE DE PROPYLENE

(30) Priorität: 23.07.2002 DE 10233388
(43) Veröffentlichungstag der Anmeldung: 04.05.2005
(73) Patentinhaber: BASF Aktiengesellschaft, 67056 Ludwigshafen (DE)
(72) Erfinder: BASSLER, Peter, 68519 Viernheim (DE); GÖBBEL, Hans-Georg, 67169 Kallstadt (DE); TELES, Joaquim, Henrique, 67166 Otterstadt (DE); RUDOLF, Peter, 68526 Ladenburg (DE)
(74) Vertreter: Hörschler, Wolfram Johannes
(86) Internationale Anmeldenummer: PCT/EP2003/007986
(87) Internationale Veröffentlichungsnummer: WO 2004/009566

(56) Entgegenhaltungen:
- EP-A- 1 127 601
- WO-A-00/07965
- WO-A-02/02544
- WO-A-02/45811

## Beschreibung

Die Erfindung betrifft ein kontinuierlich betriebenes Verfahren zur Reindestillation des bei der Propylenoxidsynthese durch Umsetzung eines Hydroperoxids mit Propylen als Lösungsmittel verwendeten Methanols unter gleichzeitiger Abtrennung der Methoxypropanole und der Schwersieder unter Verwendung einer Trennwandkolonne. Dabei wird das bei der Synthese anfallende Lösungsmittelgemisch in eine Leichtsiederfraktion, die Methanol, in eine Mittelsiederfraktion, die die Methoxypropanole als Azeotrop mit Wasser und eine Schwersiederfraktion, die Wasser enthält, aufgetrennt. In einer besonderen Ausführungsform kann die Trennwandkolonne auch in Form zweier thermisch gekoppelter Kolonnen vorliegen.

Nach den gängigen Verfahren des Standes der Technik kann Propylenoxid durch Umsetzung von Propylen mit Hydroperoxiden hergestellt werden, wobei diese Umsetzungen einstufig oder mehrstufig durchgeführt werden können.

Beispielsweise sieht das in der WO 00/07965 beschriebene mehrstufige Verfahren vor, dass die Umsetzung wenigstens die Schritte (i) bis (iii) umfasst:
(i) Umsetzung des Hydroperoxids mit Propylen unter Erhalt eines Produktgemisches, umfassend Propylenoxid und nicht umgesetztes Hydroperoxid,
(ii) Abtrennung des nicht umgesetzten Hydroperoxids aus der aus Stufe (i) resultierenden Mischung,
(iii) Umsetzung des abgetrennten Hydroperoxids aus Stufe (ii) mit Propylen.

Demgemäss findet die Umsetzung von Propylen mit dem Hydroperoxid in mindestens zwei Stufen (i) und (iii) statt, wobei das in Stufe (ii) abgetrennte Hydroperoxid erneut in die Reaktion eingesetzt wird.

Die Umsetzungen in den Stufen (i) und (iii) erfolgen dabei in zwei getrennten Reaktoren, die vorzugsweise als Festbettreaktoren ausgerüstet sind. Dabei ist es günstig, die Stufe (i) unter weitgehend isothermer und die Stufe (iii) unter adiabatischer Reaktionskontrolle durchzuführen. Es ist gleichfalls vorteilhaft, die Umsetzung heterogen zu katalysieren.

Vorzugsweise werden diese Reaktionsfolge in einem Lösungsmittel durchgeführt und als Hydroperoxid Wasserstoffperoxid verwendet. Besonders bevorzugtes Lösungsmittel ist Methanol.

Hierbei erreicht der Wasserstoffperoxid-Umsatz in Stufe (i) ca. 85 % bis 90 % und in Stufe (iii) ca. 95 % bezogen auf die Stufe (ii). In der Summe beträgt über beide Stufen der Wasserstoffperoxidumsatz ca. 99 % bei einer Propylenoxid-Selektivität von ca. 94 bis 95 %.

Wegen der hohen Selektivität der Reaktion wird dieses Verfahren auch als koppelproduktfreie Propylenoxidsynthese bezeichnet.

Das Propylenoxid muss aus einem Gemisch abgetrennt werden, das noch Methanol als Lösungsmittel, Wasser, Wasserstoffperoxid als Hydroperoxid sowie Nebenprodukte enthält. Nebenprodukte sind beispielsweise die Methoxypropanole bestehend aus 1-Methoxy-2-propanol und 2-Methoxy-1-propanol, die durch Reaktion von Propylenoxid mit Methanol entstehen. Ferner sind im Gemisch auch Propylenglykole, Hydroperoxypropanole, Acetaldehyd und Methylformiat vorhanden.

Methoxypropanole können beispielsweise als Lösungsmittel in Lacksystemen eingesetzt werden können. In der Aufarbeitung fallen sie in einem Strom an, der neben den Methoxypropanolen Methanol, Wasser und Propylenglykol enthält.

Die bisher durchgeführten Trennverfahren zur Wiedergewinnung besagter Wertstoffe wurde bisher typischerweise in Destillationskolonnen mit Seitenabzug oder in Kolonnen in Reihenschaltung durchgeführt. Diese Vorgehensweise ist aufwendig, da sie einen erhöhten energetischen und apparativen Aufwand erfordert.

Es war Aufgabe der vorliegenden Erfindung, die Reindestillation des bei der vorzugsweise koppelproduktfreien Propylenoxidsynthese durch Umsetzung eines Hydroperoxids mit Propylen als Lösungsmittel eingesetzten Methanols unter gleichzeitiger Gewinnung der Methoxypropanole und Absenkung des sonst üblichen Energiebedarfs zu optimieren. Dabei sollte das Lösungsmittel in einer Qualität erhalten werden, die die Wiederverwendbarkeit für die genannte Propylenoxidsynthese gewährleistet.

Diese Aufgabe konnte durch ein kontinuierlich betriebenes Verfahren zur Reindestillation des bei der vorzugsweise koppelproduktfreien Propylenoxidsynthese durch Umsetzung eines Hydroperoxids mit Propylen als Lösungsmittel verwendeten Methanols und der Methoxypropanole in einer Trennwandkolonne gelöst werden.

Gegenstand der Erfindung ist somit ein Verfahren zur kontinuierlich betriebenen Reindestillation des bei der Propylenoxidsynthese durch Umsetzung eines Hydroperoxids mit Propylen als Lösungsmittel verwendeten Methanols unter gleichzeitiger Abtrennung der Methoxypropanole, dadurch gekennzeichnet, dass das bei der Synthese anfallende Lösungsmittelgemisch in einer Trennwandkolonne in eine Leichtsiederfraktion, die Methanol, in eine Mittelsiederfraktion, die die Methoxypropanole als Azeotrop mit Wasser und eine Schwersiederfraktion, die Wasser und Propylenglykol enthält, aufgetrennt wird.

Erfindungsgemäß werden dabei das Methanol über den Kopf der Kolonne abdestilliert, die Methoxypropanole als Azeotrop mit Wasser über den Seitenabzug und die Schwersieder mit dem Sumpf der Trennwandkolonne entnommen.

Nach dem erfindungsgemäßen Verfahren kann das Methanol in so reiner Form gewonnen werden, dass es beispielsweise als Lösungsmittel für die Propylenoxidsynthese wiederverwendet werden kann. Auch die Methoxypropanole fallen im Gemisch als Azeotrop mit Wasser in guter Reinheit an. Im Vergleich zu den beim Stand der Technik geschilderten Verfahren führt das neue erfindungsgemäße Verfahren zu einem reduzierten apparativen Aufwand. Darüber hinaus zeichnet sich die Trennwandkolonne durch einen besonders niedrigen Energieverbrauch aus und bietet somit hinsichtlich des Energiebedarfs gegenüber einer konventionellen Kolonne oder einer Anordnung von konventionellen Kolonnen Vorteile. Für die industrielle Anwendung ist dies außerordentlich vorteilhaft.

Destillationskolonnen mit Seitenabzügen und Trennwand, im Folgenden auch als Trennwandkolonnen bezeichnet, sind bereits bekannt. Sie stellen eine Weiterentwicklung von Destillationskolonnen dar, die nur über einen Seitenabzug, jedoch über keine Trennwand verfügen. Die Anwendungsmöglichkeit des zuletzt genannten Kolonnentyps ist eingeschränkt, weil die an der Seitenabzugsstelle entnommenen Produkte nie völlig rein sind. Bei Seitenabnahmen im Verstärkungsteil der Kolonne, die üblicherweise in flüssiger Form erfolgen, enthält das Seitenprodukt noch Anteile an leichtsiedenden Komponenten, die über Kopf abgetrennt werden sollen. Bei Seitenabnahmen im Abtriebsteil der Kolonne, die meist dampfförmig erfolgen, weist das Seitenprodukt noch Hochsiederanteile auf. Die Verwendung von konventionellen Seitenabzugskolonnen ist daher auf Fälle begrenzt, in denen verunreinigte Seitenprodukte zulässig sind.
Beim Einbau einer Trennwand in eine solche Kolonne kann jedoch die Trennwirkung verbessert werden. Bei dieser Bauart ist es möglich, Seitenprodukte in reiner Form zu entnehmen. Im mittleren Bereich oberhalb und unterhalb der Zulaufstelle und der Seitenentnahmestelle ist eine Trennwand angebracht, wobei diese fest verschweißt oder auch nur gesteckt werden kann. Sie dichtet den Entnahmeteil gegenüber dem Zulaufteil ab und unterbindet in diesem Kolonnenteil eine Quervermischung von Flüssigkeits- und Brüdenströmen über den gesamten Kolonnenquerschnitt. Hierdurch verringert sich bei der Auftrennung von Vielstoffgemischen, deren Komponenten ähnliche Siedepunkte besitzen, die Zahl der insgesamt benötigten Destillationskolonnen.

Dieser Kolonnentyp wurde beispielsweise zur Trennung einer Komponentenvorlage aus Methan, Ethan, Propan und Butan verwendet (US 2,471,134), zur Trennung eines Gemisches von Benzol, Toluol und Xylol (US 4,230,533) sowie zur Trennung eines Gemisches aus n-Hexan, n-Heptan und n-Octan (EP 0 122 367).

Auch zur Trennung azeotrop siedender Mischungen können Trennwandkolonnen erfolgreich eingesetzt werden (EP 0 133 510).

Schließlich sind auch Trennwandkolonnen, in denen chemische Reaktionen unter gleichzeitiger Destillation der Produkte durchgeführt werden können, bekannt. Als Beispiele werden Veresterungen, Umesterungen, Verseifungen sowie Acetalisierungen genannt (EP 0 126 288).

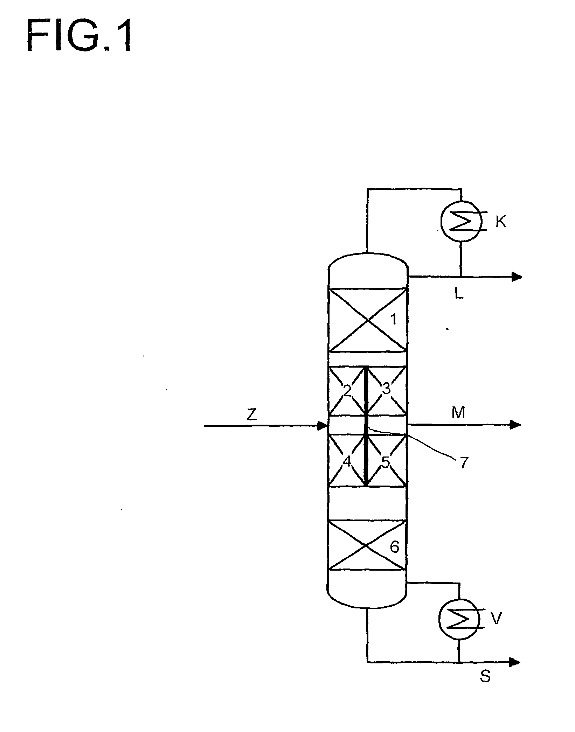

In Figur 1 ist schematisch die Reindestillation des bei der Propylenoxidsynthese als Lösungsmittel verwendeten Methanols unter gleichzeitiger Abtrennung der Methoxypropanole und der Schwersieder in einer Trennwandkolonne mit einer Seitenabzugsstelle dargestellt.

Dabei wird das aus der Propylenoxidsynthese stammende Lösungsmittelgemisch über den Zulauf Z kontinuierlich in die Trennwandkolonne eingebracht. In der Kolonne wird besagtes Gemisch aufgetrennt in die Leichtsiederfraktion L, die Methanol enthält, in die Mittelsiederfraktion M, die die Methoxypropanole als Azeotrop mit Wasser enthält, und in die Schwersiederfraktion S, die Wasser enthält.

Die Leichtsiederfraktion L wird über den Kopf der Kolonne entnommen und mit dem Kondensator K kondensiert. Am Seitenabzug für Mittelsieder M werden die Methoxypropanole als Azeotrop mit Wasser in flüssiger oder dampfförmiger Form entnommen. Zur Entnahme an der Seitenentnahmestelle eignen sich sowohl innenliegende als auch außerhalb der Kolonne angeordnete Auffangräume, in denen die Flüssigkeit oder kondensierender Dampf gesammelt werden kann. Die Schwersiederfraktion S wird mit dem Sumpf der Kolonne entnommen. Die Energiezufuhr erfolgt dabei über den Verdampfer V.

Zur Durchführung des erfindungsgemäßen Verfahrens wird eine Trennwandkolonne verwendet, die vorzugsweise 15 bis 60, besonders bevorzugt 20 bis 35, theoretische Trennstufen besitzt. Mit dieser Ausführung kann das erfindungsgemäße Verfahren besonders günstig durchgeführt werden.

Demzufolge ist das erfindungsgemäße Verfahren dadurch gekennzeichnet, dass die Trennwandkolonne 15 bis 60 theoretische Böden besitzt.

Dabei weist der obere gemeinsame Teilbereich 1 des Zulauf- und Entnahmeteils der Trennwandkolonne vorzugsweise 5 bis 50 %, besonders bevorzugt 15 bis 30 %, der Verstärkungsteil 2 des Zulaufteils vorzugsweise 5 bis 50 %, besonders bevorzugt 15 bis 30 %, der Abtriebsteil 4 des Zulaufteils vorzugsweise 5 bis 50 %, besonders bevorzugt 15 bis 30 %, der Abtriebsteil 3 des Entnahmeteils vorzugsweise 5 bis 50 %, besonders bevorzugt 15 bis 30 %, der Verstärkungsteil 5 des Entnahmeteils vorzugsweise 5 bis 50 %, besonders bevorzugt 15 bis 30 %, und der gemeinsame untere Teilbereich 6 des Zulauf- und Entnahmeteils der Trennwandkolonne vorzugsweise 5 bis 50 %, besonders bevorzugt 15 bis 30 %, der Gesamtzahl der theoretischen Trennstufen der Kolonne auf. Die Trennwand 7 verhindert die Vermischung von Flüssigkeits- und Brüdenströmen.

Vorzugsweise beträgt die Summe der Zahl der theoretischen Trennstufen der Teilbereiche 2 und 4 im Zulaufteil 80 bis 110 %, besonders bevorzugt 90 bis 100 %, der Summe der Zahl der Trennstufen der Teilbereiche 3 und 5 im Entnahmeteil.

Gleichfalls günstig ist es, die Zulaufstelle und die Seitenabzugsstelle hinsichtlich der Lage der theoretischen Trennstufen auf unterschiedlicher Höhe in der Kolonne anzuordnen. Vorzugsweise ist die Zulaufstelle um 1 bis 8, besonders bevorzugt um 3 bis 5, theoretische Trennstufen höher oder niedriger angeordnet als die Seitenabzugsstelle.

Die beim erfindungsgemäßen Verfahren verwendete Trennwandkolonne kann vorzugsweise sowohl als Packungskolonne mit Füllkörpern oder geordneten Packungen oder als Bodenkolonne ausgeführt werden. Beispielsweise können als geordnete Packungen Blech- oder Gewebepackungen mit einer spezifischen Oberfläche von 100 bis 1000 m²/m³, bevorzugt etwa 250 bis 750 m²/m³ eingesetzt werden. Solche Packungen bieten eine hohe Trennleistung bei gleichzeitig niederem Druckverlust pro Trennstufe.

Vorzugsweise wird bei vorstehend genannter Ausführung der Kolonne der durch die Trennwand unterteilte Teilbereich der Kolonne bestehend aus dem Verstärkungsteil 2 des Zulaufteils, dem Abtriebsteil 3 des Entnahmeteils, dem Abtriebsteil 4 des Zulaufteils und dem Verstärkungsteil 5 oder Teilen davon mit geordneten Packungen oder Füllkörpern bestückt, und die Trennwand 7 in diesen Teilbereichen wärmeisolierend ausgeführt.

Das zu trennende Lösungsmittelgemisch wird in Form des Zulaufstroms, der die Leicht-, Mittel- und Hochsieder enthält, über den Zulauf Z kontinuierlich in die Kolonne eingebracht. Dieser Zulaufstrom ist im allgemeinen flüssig. Es kann jedoch von Vorteil sein, den Zulaufstrom einer Vorverdampfung zu unterziehen, und anschließend zweiphasig, d. h. gasförmig und flüssig oder in Form eines gasförmigen und eines flüssigen Stromes der Kolonne zuzuführen. Diese Vorverdampfung bietet sich besonders dann an, wenn der Zulaufstrom größere Mengen an Leichtsiedern enthält. Durch die Vorverdampfung kann der Abtriebsteil der Kolonne wesentlich entlastet werden.

Zweckmäßigerweise wird der Zulaufstrom mittels einer Pumpe oder über eine statische Zulaufhöhe von mindestens 1 m mengengeregelt in den Zulaufteil aufgegeben. Vorzugsweise erfolgt diese Zugabe über eine Kaskadenregelung in Verbindung mit der Flüssigkeitsstandregelung des Auffangraumes des Zulaufteils. Dabei wird die Regelung so eingestellt, dass die auf das Verstärkungsteil 2 aufgegebene Flüssigkeitsmenge nicht unter 30 % des Normalwertes sinken kann. Es hat sich gezeigt, dass eine derartige Vorgehensweise zur Kompensation von störenden Schwankungen bezüglich der Zulaufmenge oder der Zulaufkonzentration wichtig ist.

Ähnlich wichtig ist, dass die Aufteilung der aus dem Abtriebsteil 3 des Entnahmeteils der Kolonne ablaufenden Flüssigkeit auf den Seitenabzug und auf den Verstärkungsteil 5 des Entnahmeteils durch eine Regelung so eingestellt wird, dass die auf den Teilbereich 5 aufgegebene Flüssigkeitsmenge nicht unter 30 % des Normalwertes sinken kann.

Die Einhaltung dieser Voraussetzungen muss durch entsprechende Regelvorschriften sichergestellt werden.

Regelungsmechanismen zum Betreiben von Trennwandkolonnen wurden beispielsweise beschrieben in Chem. Eng. Technol. 10 (1987) 92-98, Chem.-Ing.-Technol. 61 (1989) Nr. 1, 16-25, Gas Separation and Purification 4 (1990) 109-114, Process Engineering 2 (1993) 33-34, Trans IChemE 72 (1994) Part A 639-644, Chemical Engineering 7 (1997) 72-76). Die bei diesem Stand der Technik angegebenen Regelungsmechanismen können auch für das erfindungsgemäße Verfahren angewendet bzw. auf dieses übertragen werden.

Für die kontinuierlich betriebene Reindestillation des Lösungsmittels hat sich nachfolgend beschriebenes Regelungsprinzip als besonders günstig erwiesen. Es ist in der Lage, Lastschwankungen gut zu verkraften. Vorzugsweise erfolgt somit die Destillatentnahme temperaturgeregelt.

Im oberen Kolonnenteil 1 ist eine Temperaturregelung vorgesehen, die als Stellgröße die Ablaufmenge, das Rücklaufverhältnis oder bevorzugt die Rücklaufmenge nutzt. Dabei befindet sich die Messstelle für die Temperaturregelung vorzugsweise um drei bis acht, mehr bevorzugt um vier bis sechs, theoretische Trennstufen unterhalb des oberen Endes der Kolonne.

Durch eine geeignete Temperatureinstellung wird dann die aus dem Kolonnenteil 1 ablaufende Flüssigkeit am oberen Ende der Trennwand 7 so aufgeteilt, dass das Verhältnis des Flüssigkeitsstroms zum Zulaufteil zu dem zum Entnahmeteil vorzugsweise 0,1 bis 1,0, besonders bevorzugt 0,3 bis 0,6, beträgt.

Vorzugsweise wird bei dieser Methode die ablaufende Flüssigkeit in einem in oder außerhalb der Kolonne angeordneten Auffangraum gesammelt, woraus sie dann kontinuierlich in die Kolonne eingespeist wird. Dieser Auffangraum kann somit die Aufgabe einer Pumpenvorlage übernehmen oder für eine ausreichend hohe statische Flüssigkeitshöhe sorgen, die eine durch Stellorgane, beispielsweise Ventile, geregelte Flüssigkeitsweiterleitung ermöglicht. Bei der Verwendung von gepackten Kolonnen wird die Flüssigkeit zunächst in Sammler gefasst und von dort aus in einen innenliegenden oder außenliegenden Auffangraum geleitet.

Der Brüdenstrom am unteren Ende der Trennwand 7 wird durch die Wahl und/oder Dimensionierung der Trenneinbauten und/oder den Einbau druckmindemder Vorrichtungen, beispielsweise von Blenden, so eingestellt, dass das Verhältnis des Brüdenstroms im Zulaufteil zu dem des Entnahmeteils vorzugsweise 0.8 bis 1.2, bevorzugt 0,9 bis 1,1, beträgt.

Beim vorstehend genannten Regelprinzip ist des weiteren eine Temperaturregelung im unteren gemeinsamen Kolonnenteil 6 vorgesehen die als Stellgröße die Sumpfentnahmemenge nutzt. Somit kann die Entnahme des Sumpfprodukts temperaturgeregelt erfolgen. Dabei ist die Messstelle für die Temperaturregelung vorzugsweise um drei bis sechs, besonders bevorzugt vier bis sechs, theoretische Trennstufen oberhalb des unteren Endes der Kolonne angeordnet.

Zusätzlich kann die genannte Standregelung am Kolonnenteil 6 (Kolonnensumpf) als Stellgröße für die Seitenentnahmemenge genutzt werden. Hierzu wird als Regelgröße der Flüssigkeitsstand im Verdampfer verwendet.

Als Stellgröße der Heizleistung kann auch der Differenzdruck über die Kolonne genutzt werden. Günstigerweise wird die Destillation bei einem Kopfdruck zwischen 1 und 15 bar, bevorzugt zwischen 5 und 13 bar, durchgeführt. Dementsprechend wird zur Einhaltung dieses Druckbereiches die Heizleistung des Verdampfers am Kolonnenboden gewählt.

Dabei resultiert eine Destillationstemperatur, die vorzugsweise zwischen 30 und 140 °C, besonders bevorzugt zwischen 60 und 140 °C und insbesondere zwischen 100 und 130 °C liegt. Die Destillationstemperatur wird dabei im Kopf der Kolonne gemessen.

Demzufolge ist das erfindungsgemäße Verfahren dadurch gekennzeichnet, dass der Druck zwischen 1 und 15 bar und die Destillationstemperatur 30 bis 140 °C beträgt, jeweils gemessen im Kopf der Kolonne.

Um die Trennwandkolonne störungsfrei betreiben zu können, werden die vorstehend genannten Regelmechanismen zumeist in Kombination angewendet.

Bei der Trennung von Mehrstoffgemischen in eine Leichtsieder-, Mittelsieder- und Schwersiederfraktion existieren üblicherweise Spezifikationen über den maximal zulässigen Anteil an Leichtsiedern und Schwersiedem in der Mittelsiederfraktion. Hierbei werden entweder einzelne für das Trennproblem kritische Komponenten, sogenannte Schlüsselkomponenten, oder die Summe von mehreren Schlüsselkomponenten spezifiziert.

Die Einhaltung der Spezifikation für die Hochsieder in den Mittelsiederfraktionen wird vorzugsweise über das Aufteilungsverhältnis der Flüssigkeitsmenge am oberen Ende der Trennwand 7 geregelt. Dabei wird das Aufteilungsverhältnis so eingestellt, dass die Konzentration an Schlüsselkomponenten für die Hochsiederfraktion in der Flüssigkeit am oberen Ende der Trennwand 10 bis 80 Gew.-%, bevorzugt 30 bis 50 Gew.-%, des Wertes ausmacht, der in den Seitenabzügen erzielt werden soll. Die Flüssigkeitsaufteilung kann dann so eingestellt werden, dass bei höheren Gehalten an Schlüsselkomponenten der Hochsiederfraktion mehr und bei niedrigeren Gehalten an Schlüsselkomponenten weniger Flüssigkeit auf den Zulaufteil geleitet wird.

Entsprechend wird die Spezifikation für die Leichtsieder in der Mittelsiederfraktion durch die Heizleistung geregelt. Hierbei wird die Heizleistung im Verdampfer so eingestellt, dass die Konzentration an Schlüsselkomponenten für die Leichtsiederfraktion in der Flüssigkeit am unteren Ende der Trennwand 10 bis 80 Gew.-%, bevorzugt 30 bis 50 Gew.-%, des Wertes ausmacht, der in den Seitenabzugsprodukten erzielt werden soll. Somit wird die Heizleistung dahingehend eingestellt, dass bei höherem Gehalt an Schlüsselkomponenten der Leichtsiederfraktion die Heizleistung erhöht und bei niedrigerem Gehalt an Schlüsselkomponenten der Leichtsiederfraktion die Heizleistung verringert wird.

Die Konzentration von Leicht- und Schwersiedem in der Mittelsiederfraktion kann nach üblichen Analysemethoden ermittelt werden. Beispielsweise kann zur Detektion Infrarot-Spektroskopie verwendet werden, wobei die im Reaktionsgemisch vorliegenden Verbindungen an Hand ihrer charakteristischen Absorptionen identifiziert werden. Diese Messungen können inline direkt in der Kolonne vorgenommen werden. Bevorzugt werden jedoch gaschromatographische Methoden verwendet. Hierbei ist dann vorgesehen, dass das obere und untere Ende der Trennwand Probeentnahmemöglichkeiten aufweisen. Somit können aus der Kolonne kontinuierlich oder in zeitlichen Abständen flüssige oder gasförmige Proben entnommen und hinsichtlich ihrer Zusammensetzung untersucht werden. In Abhängigkeit von der Zusammensetzung kann dann auf die entsprechenden Regelmechanismen zurückgegriffen werden.

Es ist ein Ziel des erfindungsgemäßen Verfahrens, Methanol und die Methoxypropanole als Azeotrop mit Wasser mit einer Reinheit von vorzugsweise mindestens 95 % zur Verfügung zu stellen.

In einer speziellen Ausführung der Trennwandkolonne ist es auch möglich, Zulaufteil und Entnahmeteil, die durch die Trennwand 7 voneinander separiert sind, nicht in einer Kolonne zu vereinen, sondern räumlich voneinander zu trennen. Somit kann in dieser speziellen Ausführung die Trennwandkolonne auch aus mindestens zwei voneinander räumlich getrennten Kolonnen bestehen, die dann aber miteinander thermisch gekoppelt sein müssen.

Demzufolge ist das erfindungsgemäße Verfahren auch dadurch gekennzeichnet, dass die Trennwandkolonne in Form zweier thermisch gekoppelter Kolonnen ausgeführt ist.

Solche thermisch miteinander gekoppelten Kolonnen tauschen im Allgemeinen sowohl Dampf und Flüssigkeit miteinander aus. In speziellen Ausführungsformen ist es aber auch möglich, dass sie nur Flüssigkeit miteinander austauschen. Diese spezielle Ausführung bietet dann den Vorteil, dass die thermisch miteinander gekoppelten Kolonnen auch unter verschiedenen Drücken betrieben werden können, wobei eine noch bessere Einstellung des zur Destillation erforderlichen Temperaturniveaus möglich sein kann als bei der herkömmlichen Trennwandkolonne.

Im Allgemeinen werden besagte, thermisch gekoppelte Kolonnen so betrieben, dass die Leichtsiederfraktion und die Schwersiederfraktion aus verschiedenen Kolonnen entnommen werden. Vorzugsweise wählt man den Betriebsdruck der Kolonne, an der die Leichtsiederfraktion entnommen wird, im Allgemeinen um etwa 0,5 bis 3 bar höher als den Druck in der Kolonne, an der die Schwersiederfraktion entnommen wird.

Ferner kann es bei gekoppelten Kolonnen günstig sein, Sumpfströme in einem zusätzlichen Verdampfer teilweise oder ganz zu verdampfen und erst danach der nächsten Kolonne zuzuführen. Diese Vorverdampfung bietet sich insbesondere dann an, wenn der Sumpfstrom der ersten Kolonne größere Mengen an Mittelsieder enthält. In diesem Fall kann die Vorverdampfung auf einem niedrigerem Temperaturniveau erfolgen und der Verdampfer der zweiten Kolonne entlastet werden, sofern diese Kolonne mit einem Verdampfer ausgerüstet ist. Weiterhin wird durch diese Maßnahme der Abtriebsteil der zweiten Kolonne wesentlich entlastet. Der vorverdampfte Strom kann dabei der nachfolgenden Kolonne zweiphasig oder in Form von zwei separaten Strömen zugeführt werden.

Umgekehrt ist es aber auch möglich, leichtsiedende Kopfströme teilweise oder ganz zu kondensieren, bevor sie der nächsten Kolonne zugeführt werden. Auch diese Maßnahme kann dazu beitragen, eine bessere Trennung zwischen Leichtsiedern und darin enthaltenen Mittelsiedern herbeizuführen.

In weiteren Ausführungsformen ist das erfindungsgemäße Verfahren dann auch dadurch gekennzeichnet, dass der aus einer der gekoppelten Kolonnen entnommene flüssige Sumpfstrom teilweise oder ganz verdampft wird, bevor er der anderen Kolonne zugeführt wird, und/oder der aus einer der gekoppelten Kolonnen entnommene dampfförmige Kopfstrom teilweise oder ganz kondensiert wird, bevor er der anderen Kolonne zugeführt wird.

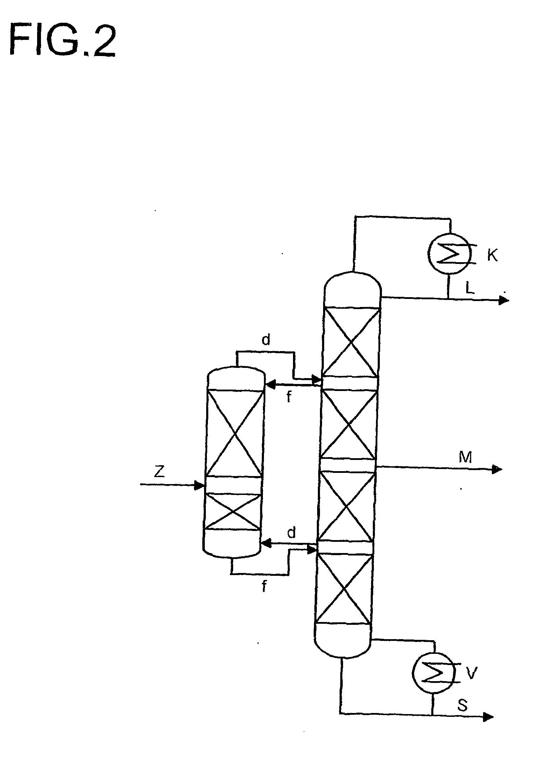

Beispiele für Trennwandkolonnen in der speziellen Ausführung der thermisch gekoppelten Kolonnen sind schematisch in den Figuren 2, 3, 4 und 5 dargestellt. Erfindungsgemäß können mit diesen Anordnungen die Leichtsiederfraktion L, enthaltend Methanol, die Mittelsiederfraktion M, enthaltend die Methoxypropanole als Azeotrop mit Wasser, sowie die Schwersiederfraktion S, enthaltend Wasser aber auch Propylenglykol, voneinander getrennt werden.

Figur 2 zeigt zwei miteinander thermisch gekoppelte Kolonnen, wobei die Kolonne, über die der Zulauf Z erfolgt, sowohl über den Kopf wie auch über den Boden Dampf d und Flüssigkeit f mit der nachgeschalteten Kolonne austauscht. Die Energiezufuhr erfolgt im Wesentlichen über den Verdampfer V der der Zulaufkolonne nachgeschalteten Kolonne. Hierbei können über den Kopf der nachgeschalteten Kolonne durch Kondensation mit dem Kondensator K die Leichtsiederfraktion L, aus dem Seitenabzug die Mittelsiederfraktion M sowie mit dem Sumpf die Schwersiederfraktion S erhalten werden.

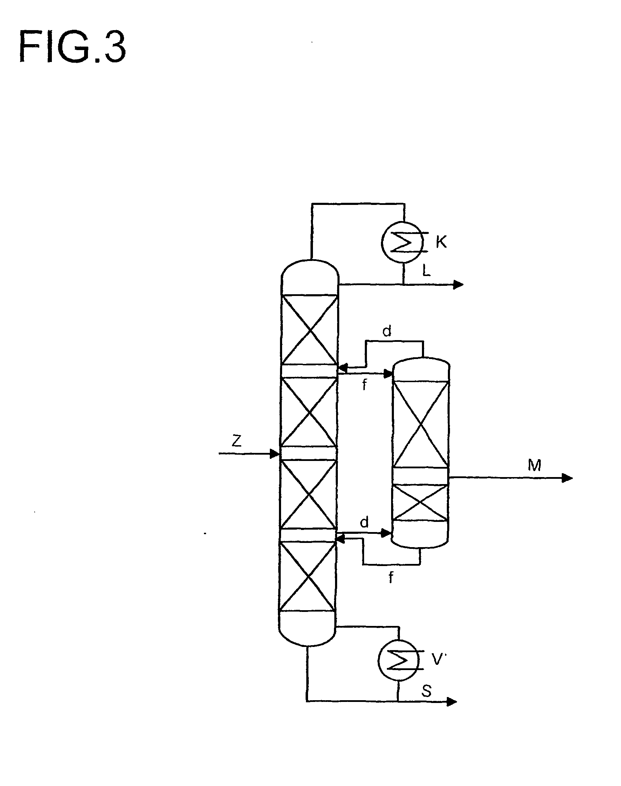

Möglich ist auch eine Verschaltung wie in Figur 3 skizziert. Hierbei werden bereits in der Zulauflcolonne über den Kopf die Leichtsiederfraktion L und mit dem Sumpf die Schwersiederfraktion S abgetrennt. Aus dem Seitenabzug der nachgeschalteten Kolonne wird die Mittelsiederfraktion M erhalten. Die nachgeschaltete Kolonne kann dabei über Kopf und Boden Dampf d und Flüssigkeit f mit der Zulaufkolonne austauschen. Die Energiezufuhr erfolgt dabei im Wesentlichen über den Verdampfer der Zulaufkolonne.

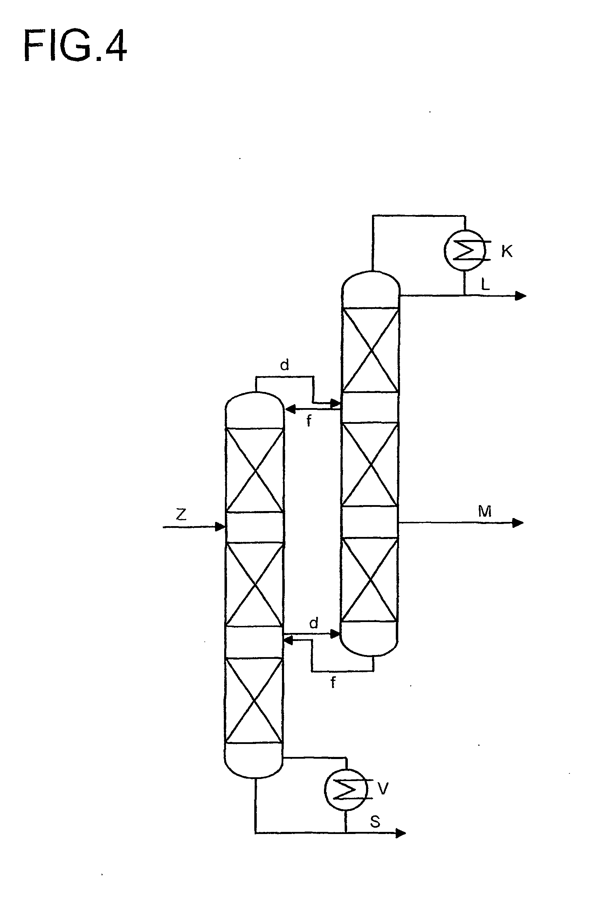

Figur 4 zeigt eine Anordnung, in der die Schwersiederfraktion S mit dem Sumpf der Zulaufkolonne erhalten wird. Die Leichtsiederfraktion L wird über den Kopf und die Mittelsiederfraktion M über den Seitenabzug der nachgeschalteten Kolonne erhalten. Die Energiezufuhr erfolgt dabei im Wesentlichen über den Verdampfer der Zulaufkolonne.

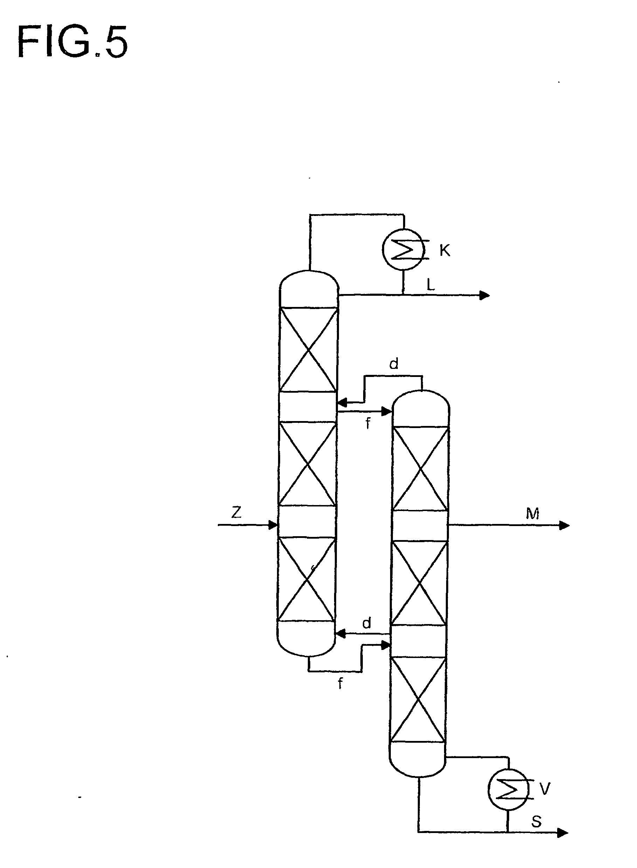

Figur 5 zeigt eine Anordnung, in der die Leichtsiederfraktion L über den Kopf der Zulaufkolonne erhalten wird. In der nachgeschalteten Kolonne wird mit dem Sumpf die Schwersiederfraktion S und über den Seitenabzug die Mittelsiederfraktion M erhalten. Die Energiezufuhr erfolgt dabei im Wesentlichen über den Verdampfer der der Zulaufkolonne nachgeschalteten Kolonne.

Somit ist das erfindungsgemäße Verfahren auch dadurch gekennzeichnet, dass das Lösungsmittelgemisch in der der Zulaufkolonne nachgeschalteten Kolonne in die Leicht-, Mittel- und Schwersiederfraktion aufgetrennt wird, oder
dass das Lösungsmittelgemisch in der Zulaufkolonne in die Leicht- und Schwersiederfraktion und in der nachgeschalteten Kolonne in die Mittelsiederfraktion aufgetrennt wird, oder
dass das Lösungsmittelgemisch in der Zulaufkolonne in die Schwersiederfraktion und in der nachgeschalteten Kolonne in die Leicht- und Mittelsiederfraktion aufgetrennt wird, oder
dass das Lösungsmittelgemisch in der Zulaufkolonne in die Leichtsiederfraktion und in der nachgeschalteten Kolonne in die Mittel- und Schwersiederfraktion aufgetrennt wird.

Auch die Kolonnen nach Fig. 2 bis 5 können als Packungskolonnen mit Füllkörpern oder geordneten Packungen oder als Bodenkolonnen ausgeführt werden. Beispielsweise können als geordnete Packungen Blech- oder Gewebepackungen mit einer spezifischen Oberfläche von 100 bis 1000 m²/m³, bevorzugt etwa 250 bis 750 m²/m³ eingesetzt werden. Solche Packungen bieten eine hohe Trennleistung bei gleichzeitig niederem Druckverlust pro Trennstufe.

Für das erfindungsgemäße Verfahren können zur Herstellung des Propylenoxids die aus dem Stand der Technik bekannten Edukte verwendet werden.

Propylen kann in der Qualitätsstufe "chemical grade" eingesetzt werden. Ein solches Propylen enthält Propan, wobei Propylen und Propan im Volumenverhältnis von ca. 97 : 3 bis 95 : 5 vorliegen.

Als Hydroperoxid können die bekannten Hydroperoxide, die für die Umsetzung der organischen Verbindung geeignet sind, eingesetzt werden. Beispiele für solche Hydroperoxide sind etwa tert.-Butylhydroperoxid oder Ethylbenzolhydroperoxid. Bevorzugt wird als Hydroperoxid für die Oxiransynthese Wasserstoffperoxid eingesetzt, wobei auch eine wässerige Wasserstoffperoxidlösung verwendet werden kann.

Wasserstoffperoxid kann beispielsweise über das Anthrachinon-verfahren, wie es in "Ullmann's Encyclopedia of Industrial Chemistry", 5. Auflage, Band 13, Seiten 447 bis 456, beschrieben ist, hergestellt werden.

Ebenso ist es denkbar, zur Wasserstoffperoxidgewinnung Schwefelsäure durch anodische Oxidation unter gleichzeitiger kathodischer Wasserstoffentwicklung in Peroxodischwefelsäure zu überführen. Die Hydrolyse der Peroxodischwefelsäure führt dann auf dem Weg über Peroxoschwefelsäure zu Wasserstoffperoxid und Schwefelsäure, die damit zurückgewonnen wird.

Möglich ist selbstverständlich auch die Darstellung von Wasserstoffperoxid aus den Elementen.

Das zur Umsetzung als Lösungsmittel verwendete Methanol kann in der üblichen technischen Qualität verwendet werden und ist dann vorzugsweise mindestens 95-prozentig.

Als Katalysatoren für die Propylenoxidherstellung werden bevorzugt solche verwendet, die ein poröses oxidisches Material, wie z. B. einen Zeolith, umfassen. Vorzugsweise werden Katalysatoren eingesetzt, die als poröses oxidisches Material einen Titan-, Germanium-, Tellur-, Vanadium-, Chrom-, Niob- oder Zirkoniumhaltigen Zeolith umfassen.

Dabei sind im einzelnen Titan-, Germanium-, Tellur-, Vanadium-, Chrom-, Niob-, Zirkonium-haltige Zeolithe mit Pentasil-Zeolith-Struktur, insbesondere die Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA-, BIK-, BOG-, BPH-, BRE-, CAN-, CAS-, CFI-, CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI-, MFS-, MON-, MOR-, MSO-, MTF-, MTN-, MTT-, MTW-, MWW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WIE-, WEN-, YUG-, ZON-Struktur sowie zu Mischstrukturen aus zwei oder mehr der vorgenannten Strukturen zu nennen. Denkbar sind für den Einsatz im erfindungsgemäßen Verfahren weiterhin titanhaltige Zeolithe mit der Struktur des ITQ-4, SSZ-24, TTM-1, UTD-1, CIT-1 oder CIT-5. Als weitere titanhaltige Zeolithe sind solche mit der Struktur des ZSM-48 oder ZSM-12 zu nennen.

Als besonders bevorzugt sind Ti-Zeolithe mit MFI-, MEL- oder MFI/MEL-Mischstruktur anzusehen. Ganz besonders bevorzugt sind im Einzelnen die Titanenthaltenden Zeolith-Katalysatoren, die im allgemeinen als "TS-1", "TS-2", "TS-3" bezeichnet werden, sowie Ti-Zeolithe mit einer zu β-Zeolith isomorphen Gerüststruktur.

Sehr günstig ist die Verwendung eines heterogenen Katalysator, der das Titan-haltige Silikalit TS-1 umfasst.

Dabei ist es auch möglich, als Katalysator das poröse oxidische Material an sich zu verwenden. Selbstverständlich ist es jedoch auch möglich, als Katalysator einen Formkörper einzusetzen, der das poröse oxidische Material umfasst. Dabei können zur Herstellung des Formkörpers, ausgehend von dem porösen oxidischen Material, alle Verfahren gemäß dem Stand der Technik eingesetzt werden.

Vor, während oder nach dem einen oder mehreren Formgebungsschritten in diesen Verfahren können auf das Katalysatormaterial Edelmetalle in Form geeigneter E-delmetallkomponenten, beispielsweise in Form von wasserlöslichen Salzen aufgebracht werden. Vorzugsweise wird dieses Verfahren angewendet, um Oxidationskatalysatoren auf der Basis von Titan- oder Vanadiumsilikaten mit Zeolithstruktur herzustellen, wobei Katalysatoren erhältlich sind, die einen Gehalt von 0,01 bis 30 Gew.-% an einem oder mehreren Edelmetallen aus der Gruppe Ruthenium, Rhodium, Palladium, Osmium, Iridium, Platin, Rhenium, Gold und Silber aufweisen. Derartige Katalysatoren sind beispielsweise in der DE-A 196 23 609.6 beschrieben.

Selbstverständlich können die Formkörper konfektioniert werden. Sämtliche Verfahren zur Zerkleinerung sind dabei denkbar, beispielsweise durch Splittung oder Brechen der Formkörper, ebenso wie weitere chemische Behandlungen, wie beispielsweise vorstehend beschrieben.

Bei Verwendung eines Formkörpers oder auch mehr davon als Katalysator kann dieser im erfindungsgemäßen Verfahren nach erfolgter Deaktivierung durch ein Verfahren regeneriert werden, bei dem die Regenerierung durch gezieltes Abbrennen der für die Deaktivierung verantwortlichen Beläge erfolgt. Dabei wird bevorzugt in einer Inertgasatmosphäre gearbeitet, die genau definierte Mengen an Sauerstoff liefernden Substanzen enthält. Dieses Regenerierungsverfahren ist in der DE-A 197 23 949.8 beschrieben. Ferner können die dort bezüglich des Standes der Technik angegebenen Regenerierungsverfahren eingesetzt werden.

Im Allgemeinen liegt die Reaktionstemperatur für die Herstellung des Propylenoxids in den Stufen (i) und (iii) im Bereich von 0 bis 120 °C, bevorzugt im Bereich von 10 bis 100 °C und weiter bevorzugt im Bereich von 20 bis 90 °C. Die auftretenden Drücke reichen dabei von 1 bis 100 bar, vorzugsweise 1 bis 40 bar, mehr bevorzugt 1 bis 30 bar. Bevorzugt wird bei Drücken gearbeitet, unter denen keine Gasphase vorliegt.

Die Konzentration von Propylen und Wasserstoffperoxid im Eduktstrom wird im Allgemeinen so gewählt, dass das molare Verhältnis bevorzugt im Bereich von 0,7 bis 20, weiter bevorzugt im Bereich von 0,8 bis 5,0, besonders bevorzugt im Bereich von 0,9 bis 2,0 und insbesondere im Bereich von 1,0 bis 1,6 liegt.

Bei der Propylenoxidherstellung richten sich die Verweilzeiten im Reaktor bzw. in den Reaktoren dabei im Wesentlichen nach den gewünschten Umsätzen. Im Allgemeinen liegen sie bei weniger als 5 Stunden, bevorzugt bei weniger als 3 Stunden, weiter bevorzugt bei weniger als 1 Stunde und besonders bevorzugt bei etwa einer halben Stunde.

Als Reaktoren für die Propylenoxid-Synthese können selbstverständlich alle denkbaren, für die jeweiligen Reaktionen am besten geeigneten Reaktoren eingesetzt werden. Dabei ist ein Reaktor nicht auf einen einzelnen Behälter beschränkt. Vielmehr ist es auch möglich, beispielsweise eine Rührkesselkaskade einzusetzen.

Bevorzugt werden für die Propylenoxid-Synthese als Reaktoren Festbettreaktoren verwendet. Weiter bevorzugt werden als Festbettreaktoren Festbettrohrreaktoren eingesetzt.

Insbesondere wird für vorstehend beschriebene und bevorzugt verwendete Propylenoxidsynthese als Reaktor für die Stufe (i) ein isothermer Festbettreaktor und für die Stufe (iii) ein adiabatischer Festbettreaktor verwendet, wobei in der Stufe (ii) das Hydroperoxid in einer Abtrennvorrichtung abgetrennt wird.

Die Erfindung wird durch nachfolgendes Beispiel beschrieben.

### Beispiel

Nach dem in der WO 00/07965 angegebenen Verfahren wurde ausgehend von Propylen durch Umsetzung mit Wasserstoffperoxid Propylenoxid hergestellt, wobei die Umsetzung in Methanol als Lösungsmittel durchgeführt wurde. Das nach der Abtrennung des Propylenoxids erhaltene und aufzuarbeitende Lösungsmittelgemisch, das Methanol und die Methoxypropanole enthielt, wies folgende Zusammensetzung auf:
ca. 80,0 Gew.-% Methanol,
ca. 5,0 Gew.-% Methoxypropanole, und
ca. 15,0 Gew.-% Wasser und Propylenglykol.

Es war das Ziel der Aufarbeitung, Methanol und die Methoxypropanole (als Azeotrop mit Wasser) mit möglichst geringem energetischen Aufwand voneinander zu trennen. Dabei sollte das Methanol in einer Reinheit von wenigstens 95 % anfallen.

Dazu wurde das Gemisch mit Hilfe einer Trennwandkolonne mit einem Seitenabzug destilliert, wobei Methanol über den Kopf der Kolonne, die Methoxypropanole als Azeotrop mit Wasser aus dem Seitenabzug und Wasser im Gemisch mit Propylenglykol mit dem Sumpf der Kolonne entnommen wurden.

Der benötigte Energieinhalt der Destillation wurde als Maß für die Effektivität der Trennung eingesetzt. Er wurde berechnet aus der Verdampferleistung bezogen auf den pro Stunde durch die Kolonne erfolgten Durchsatz an aufzutrennendem Gemisch. Als Kolonnenverschaltungen wurden die in der Tabelle aufgeführten Anordnungen gewählt:

| Kolonnenverschaltung | Energiebedarf/(kg/h) [kW/(kg/h)] | Energieeinsparung [%] |
|---|---|---|
| Konventionelle Kolonne mit Seitenabzug | 0,98 | - |
| Reihenschaltung zweier konventioneller Kolonnen | 0,89 | 9,2 |
| Trennwandkolonne | 0,73 | 25,5 |

Es wird deutlich, dass die Trennwandverschaltung energetisch einen erheblichen Vorteil gegenüber der konventionellen Destillationsanordnung besaß, da der für die Destillation erforderliche Energieaufwand wesentlich niedriger lag als bei der Destillation mit den beiden konventionellen Destillationsanordnungen mit der konventionellen Kolonne mit Seitenabzug und der Reihenschaltung zweier konventioneller Kolonnen.

Das durch Destillation in der Trennwandkolonne erhaltene Methanol konnte erneut für die Propylenoxidsynthese verwendet werden.

### Bezugszeichenliste für Figuren 1 bis 5:

- 1: gemeinsamer Teilbereich des Zulauf- und Entnahmeteils der Trennwandkolonne
- 2: Verstärkungsteil des Zulaufteils
- 3: Abtriebsteil des Entnahmeteils
- 4: Abtriebsteil des Zulaufteils
- 5: Verstärkungsteil des Entnahmeteils
- 6: gemeinsamer Teilbereich des Zulauf- und Entnahmeteils
- 7: Trennwand

- Z: Zulauf
- L: Leichtsiederfraktion (Methanol)
- M: Mittelsieder (1-Methoxy-2-propanol und 2-Methoxy-1-propanol als Azeotrop mit Wasser)
- S: Schwersieder
- K: Kondensator
- V: Verdampfer

- d: Dampf
- f: Flüssigkeit

Waagrechte und diagonale oder diagonal angedeutete Linien in den Kolonnen symbolisieren Packungen mit Füllkörpern oder geordnete Packungen, die in der Kolonne vorhanden sein können.

## Patentansprüche

1. Verfahren zur kontinuierlich betriebenen Reindestillation des bei der Propylenoxidsynthese durch Umsetzung eines Hydroperoxids mit Propylen als Lösungsmittel verwendeten Methanols unter gleichzeitiger Abtrennung der Methoxypropanole, **dadurch gekennzeichnet, dass** das bei der Synthese anfallende Lösungsmittelgemisch in einer Trennwandkolonne in eine Leichtsiederfraktion, die Methanol, in eine Mittelsiederfraktionen, die die Methoxypropanole als Azeotrop mit Wasser und eine Schwersiederfraktion, die Wasser und Propylenglykol enthält, aufgetrennt wird.

2. Verfahren nach Anspruch 1, **dadurch gekennzeichnet, dass** Methanol über den Kopf der Kolonne, die Methoxypropanole als Azeotrop mit Wasser dem Seitenabzug der Kolonne sowie Wasser mit dem Sumpf entnommen werden.

3. Verfahren nach Anspruch 1 oder 2, **dadurch gekennzeichnet, dass** die Trennwandkolonne 15 bis 60 theoretische Trennstufen besitzt.

4. Verfahren nach einem der Ansprüche 1 bis 3, **dadurch gekennzeichnet, dass** der Druck 1 bis 15 bar und die Destillationstemperatur 30 bis 140 °C beträgt, jeweils gemessen im Kopf der Kolonne.

5. Verfahren nach einem der Ansprüche 1 bis 4, **dadurch gekennzeichnet, dass** die Trennwandkolonne in Form zweier thermisch gekoppelter Kolonnen ausgeführt ist.

6. Verfahren nach Anspruch 5, **dadurch gekennzeichnet, dass** das Lösungsmittelgemisch in der der Zulaufkolonne nachgeschalteten Kolonne in die Leicht-, Mittel- und Schwersiederfraktion aufgetrennt wird, oder
dass das Lösungsmittelgemisch in der Zulaufkolonne in die Leicht- und Schwersiederfraktion und in der nachgeschalteten Kolonne in die Mittelsiederfraktion aufgetrennt wird, oder
dass das Lösungsmittelgemisch in der Zulaufkolonne in die Schwersiederfraktion und in der nachgeschalteten Kolonne in die Leicht- und Mittelsiederfraktion aufgetrennt wird, oder
dass das Lösungsmittelgemisch in der Zulaufkolonne in die Leichtsiederfraktion und in der nachgeschalteten Kolonne in die Mittel- und Schwersiederfraktion aufgetrennt wird.

7. Verfahren nach Anspruch 5 oder 6, **dadurch gekennzeichnet, dass** der aus einer der gekoppelten Kolonnen entnommene flüssige Sumpfstrom teilweise oder ganz verdampft wird, bevor er der anderen Kolonne zugeführt wird, und der aus einer der gekoppelten Kolonnen entnommene dampfförmige Kopfstrom teilweise oder ganz kondensiert wird, bevor er der anderen Kolonne zugeführt wird.

8. Verfahren nach Anspruch 5 oder 6, **dadurch gekennzeichnet, dass** der aus einer der gekoppelten Kolonnen entnommene Sumpfstrom teilweise oder ganz verdampft wird, bevor er der anderen Kolonne zugeführt wird, oder der aus einer der gekoppelten Kolonnen entnommene Kopfstrom teilweise oder ganz kondensiert wird, bevor er der anderen Kolonne zugeführt wird.

9. Verfahren nach einem der Ansprüche 1 bis 8, **dadurch gekennzeichnet, dass** das Propylenoxid hergestellt wird durch ein Verfahren umfassend wenigstens die Schritte (i) bis (iii):
(i) Umsetzung des Hydroperoxids mit Propylen,
(ii) Abtrennung des nicht umgesetzten Hydroperoxids aus der aus Stufe
(i) resultierenden Mischung,
(iii) Umsetzung des abgetrennten Hydroperoxids aus Stufe (ii) mit Propylen,
wobei in Stufe (i) ein isothermer Festbettreaktor, in Stufe (iii) ein adiabatischer Festbettreaktor, in Stufe (ii) eine Abtrennvorrichtung und als Hydroperoxid Wasserstoffperoxid verwendet werden sowie die organische Verbindung während der Reaktion in Kontakt mit einem heterogenen Katalysator gebracht wird.

10. Verfahren nach Anspruch 9, **dadurch gekennzeichnet, dass** der heterogene Katalysator den Zeolith TS-1 umfasst.

## Claims

1. A process for the continuously operated purification by distillation of the methanol used as solvent in the synthesis of propylene oxide by reaction of a hydroperoxide with propylene, with the methoxypropanols being separated off simultaneously, wherein the solvent mixture obtained in the synthesis is separated in a dividing wall column into a low-boiling fraction comprising methanol, an intermediate-boiling fraction comprising the methoxypropanols as azeotrope with water and a high-boiling fraction comprising water and propylene glycol.

2. The process according to claim 1, wherein methanol is taken off via the top of the column, the methoxypropanols are taken off as azeotrope with water from the side offtake of the column and water is taken off at the bottom.

3. The process according to claim 1 or 2, wherein the dividing wall column has from 15 to 60 theoretical plates.

4. The process according to any of claims 1 to 3, wherein the pressure is from 1 to 15 bar and the distillation temperature is from 30 to 140°C, in each case measured at the top of the column.

5. The process according to any of claims 1 to 4, wherein the dividing wall column is configured as two thermally coupled columns.

6. The process according to claim 5, wherein the solvent mixture is separated into the low-boiling, intermediate-boiling and high-boiling fractions in the column downstream of the feed column, or
the low-boiling and high-boiling fractions are taken off from the solvent mixture in the feed column and the intermediate-boiling fraction is taken off in the downstream column, or
the high-boiling fraction is taken off from the solvent mixture in the feed column and the low-boiling and intermediate-boiling fractions are taken off in the downstream column, or
the low-boiling fraction is taken off from the solvent mixture in the feed column and the intermediate-boiling and high-boiling fractions are taken off in the downstream column.

7. The process according to claim 5 or 6, wherein the liquid stream taken from the bottom of one of the coupled columns is partly or completely vaporized before it is passed to the other column, and the gaseous stream taken off at the top of one of the coupled columns is partly or completely condensed before it is passed to the other column.

8. The process according to claim 5 or 6, wherein the stream taken from the bottom of one of the coupled columns is partly or completely vaporized before it is passed to the other column, or the stream taken off at the top of one of the coupled columns is partly or completely condensed before it is passed to the other column.

9. The process according to any of claims 1 to 8, wherein the propylene oxide is prepared by a process comprising at least the steps (i) to (iii)
(i) reaction of the hydroperoxide with propylene,
(ii) separation of the unreacted hydroperoxide from the mixture resulting from step (i),
(iii) reaction of the hydroperoxide which has been separated off in step (ii) with propylene,
with an isothermal fixed-bed reactor being used in step (i), an adiabatic fixed-bed reactor being used in step (iii), a separation apparatus being used in step (ii) and hydrogen peroxide being used as hydroperoxide and the organic compound being brought into contact with a heterogeneous catalyst during the reaction.

10. The process according to claim 9, wherein the heterogeneous catalyst comprises the zeolite TS-1.

## Revendications

1. Procédé de distillation continue à l'état pur du méthanol utilisé comme solvant lors de la synthèse d'oxyde de propylène par réaction d'un hydroperoxyde avec du propylène, avec séparation simultanée des méthoxypropanols, **caractérisé en ce que** le mélange de solvants obtenu lors de la synthèse est séparé dans une colonne à paroi séparatrice en une fraction à bas point d'ébullition, le méthanol, une fraction à point d'ébullition moyen, qui contient les méthoxypropanols sous la forme d'un azéotrope avec l'eau, et une fraction à haut point d'ébullition qui contient de l'eau et du propylèneglycol.

2. Procédé suivant la revendication 1, **caractérisé en ce que** du méthanol est prélevé par la tête de la colonne, les méthoxypropanols sous la forme d'un azéotrope avec l'eau par un soutirage latéral de la colonne et de l'eau par le fond.

3. Procédé suivant la revendication 1 ou 2, **caractérisé en ce que** la colonne à paroi séparatrice possède 15 à 60 étages séparateurs théoriques.

4. Procédé suivant l'une des revendications 1 à 3, **caractérisé en ce que** la pression est de 1 à 15 bars et la température de distillation de 30 à 140°C, chacune mesurée dans la tête de la colonne.

5. Procédé suivant l'une des revendications 1 à 4, **caractérisé en ce que** la colonne à paroi séparatrice est réalisée sous la forme de deux colonnes thermiquement couplées.

6. Procédé suivant la revendication 5, **caractérisé en ce que** le mélange de solvants est séparé en les fractions à bas, moyen et haut point d'ébullition dans la colonne montée en aval de la colonne d'alimentation, ou
**en ce que** le mélange de solvants est séparé en les fractions à bas et haut point d'ébullition dans la colonne d'alimentation et en la fraction à point d'ébullition moyen dans la colonne montée en aval, ou
**en ce que** le mélange de solvants est séparé en la fraction à haut point d'ébullition dans la colonne d'alimentation et en les fractions à bas point d'ébullition et à point d'ébullition moyen dans la colonne montée en aval, ou
**en ce que** le mélange de solvants est séparé en la fraction à bas point d'ébullition dans la colonne d'alimentation et en les fractions à point d'ébullition moyen et à haut point d'ébullition dans la colonne montée en aval.

7. Procédé suivant la revendication 5 ou 6, **caractérisé en ce que** le courant liquide de fond, prélevé d'une des colonnes couplées, est partiellement ou totalement évaporé avant d'être amené à l'autre colonne et **en ce que** le courant de tête en forme de vapeur, prélevé d'une des colonnes couplées, est partiellement ou totalement condensé avant d'être amené à l'autre colonne.

8. Procédé suivant la revendication 5 ou 6, **caractérisé en ce que** le courant de fond prélevé d'une des colonnes couplées est partiellement ou totalement évaporé avant d'être amené à l'autre colonne ou **en ce que** le courant de tête prélevé d'une des colonnes couplées est partiellement ou totalement condensé avant d'être amené à l'autre colonne.

9. Procédé suivant l'une des revendications 1 à 8, **caractérisé en ce qu'**on prépare l'oxyde de propylène par un procédé comprenant au moins les étapes (i) à (iii) :
(i) une réaction de l'hydroperoxyde avec du propylène,
(ii) une séparation de l'hydroperoxyde n'ayant pas réagi à partir du mélange résultant de l'étape (i),
(iii) une réaction de l'hydroperoxyde séparé issu de l'étape (ii) avec du propylène,
un réacteur à lit fixe isotherme étant utilisé dans l'étape (i), un réacteur à lit fixe adiabatique dans l'étape (iii), un dispositif de séparation dans l'étape (ii) et du peroxyde d'hydrogène comme hydroperoxyde, le composé organique étant amené en contact avec un catalyseur hétérogène pendant la réaction.

10. Procédé suivant la revendication 9, **caractérisé en ce que** le catalyseur hétérogène comporte la zéolite TS-1.
